# FASCICULE DE BREVET EUROPEEN

(11) **EP 3 151 763 B1**
(45) Date de publication et mention de la délivrance du brevet: **12.09.2018**
(21) Numéro de dépôt: 15733805.4
(22) Date de dépôt: 04.06.2015
(51) Int. Cl.: A61B 17/3207, A61B 17/22, A61F 2/24

(54) **DISPOSITIF TRANSCATHETER POUR L'ABLATION DE TISSUS CALCIFIES AU NIVEAU DES VOLETS D'UNE VALVE AORTIQUE**
KATHETERVORRICHTUNG ZUR ABLATION VON VERKALKTEM GEWEBE AN DEN KLAPPEN EINER AORTENKLAPPE
TRANSCATHETER DEVICE FOR THE ABLATION OF CALCIFIED TISSUE AT THE FLAPS OF AN AORTIC VALVE

(30) Priorité: 05.06.2014 FR 1455146
(43) Date de publication de la demande: 12.04.2017
(73) Titulaire: AORTICLAB SARL, 1073 Savigny (CH); Centre Hospitalier Universitaire De Saint Etienne, 42055 Saint-Etienne Cedex 2 (FR)
(72) Inventeur: PAIN, Bernard, 43120 Monistrol-sur-Loire (FR); VOLA, Marco, 42270 Saint-Priest-en Jarez (FR); PASQUINO, Enrico, 1073 Savigny (IT)
(74) Mandataire: Grosfillier, Philippe
(86) Numéro de dépôt international: PCT/FR2015/051491
(87) Numéro de publication internationale: WO 2015/185872

(56) Documents cités:
- WO-A2-03/088809
- FR-A1- 2 882 916
- US-A1- 2004 049 215

## Description

L'invention concerne la technique de la chirurgie et de la cardiologie interventionnelle, et concerne plus particulièrement un dispositif transcathéter pour l'ablation de tissus calcifiés, notamment de valves cardiaques.

Il est parfaitement connu que la pathologie sévère des valves aortiques résulte principalement d'une calcification dégénérative de la valve laquelle calcification s'accroit avec l'âge. Ainsi, lorsque les valves deviennent sévèrement sténotiques et cliniquement symptomatiques, il est nécessaire de réaliser une intervention chirurgicale pour le remplacement de la valve ou pour une implantation de la valve par voie transcathéter, par exemple connue sous le nom de TAVI.

Brevet US 2004/049215 A1, divulgue un exemple d'un dispositif trans-cathéter connu, pour l'ablation de tissus calcifiés. Il a été constaté que l'implantation d'une valve aortique par TAVI à travers une valve aortique calcifiée, génère très souvent des fuites para-valvulaires en cas de concentration excessive et mal équilibrée des noyaux de calcium. Ces fuites peuvent avoir un impact sur la durée de vie du patient. Une autre complication de la présence du calcium pendant le TAVI est l'embolisation, qui peut être coronarienne, générant un infarctus périprocédure, ou cérébral qui conduit à des AVC iatrogènes causés par le TAVI et qui sont une des limites de cette technique.

Des essais cliniques ont également démontré qu'au moins 10 % des patients présentent un état de valve aortique bicuspide lors d'un électrocardiogramme. La morphologie d'une valve bicuspide, est une condition de limitation de la réussite d'un TAVI. En effet, dans le cas d'une valve aortique bicuspide, la minéralisation des tissus est asymétrique, avec un taux de calcification valvulaire et annulaire très élevé et mal distribué comparativement aux autres valves aortiques. Il résulte que l'implantation d'un TAVI, dans de telles conditions, est affectée par plusieurs inconvénients cliniques majeurs comme l'apparition de fuites péri-valvulaires qui peuvent être importantes ou la migration de l'implant TAVI.

Afin d'obtenir de bons résultats par TAVI, il est très important de poser la valve sur une surface la plus régulière possible afin d'éviter une distorsion susceptible de rendre l'ouverture de la prothèse incompatible, avec pour objectif de minimiser les fuites péri-valvulaires qui affectent de différentes manières au moins 80 % des TAVI.

A partir de l'analyse de cet état de la technique, un des problèmes que se propose de résoudre l'invention est de créer un dispositif apte à enlever les tissus calcifiés et végétations dans et dessus les volets de la valve aortique par l'implantation d'une valve aortique par TAVI. Le but recherché est donc, à travers une approche transcathéter, de permettre une ablation partielle ou totale du calcium des valves pour une optimisation de la surface en vue de l'implantation de la valve.

Pour résoudre ce problème et atteindre ces objectifs, il a été conçu et mis au point un dispositif transcathéter pour l'ablation de tissus calcifiés au niveau des volets d'une valve aortique, lequel dispositif comprend un corps souple faisant office de cathéter et présentant un embout souple et flexible coopérant avec un guide-fil préalablement introduit et apte à traverser les volets de la valve au-dessus de la partie où doivent être enlevés les tissus calcifiés, ledit embout présentant au moins un système de coupe monté en combinaison avec un moyen d'aspiration sous-vide, ledit embout étant équipé d'un moyen de guidage réglable apte à coopérer avec les tissus calcifiés au fur et à mesure de l'opération d'ablation par le système de coupe en combinaison avec un effet de trajectoire en spirale appliqué à l'embout.

Il ressort des caractéristiques de l'invention que le dispositif d'ablation transcathéter est adapté pour être introduit directement de la racine aortique avec une ponction directe de l'aorte ascendante, avec par exemple, un accès transaortique par l'intermédiaire d'une petite thoracotomie, ou par un trocart d'endoscopie, ou par un accès transcathéter via l'artère fémorale ou par un accès via d'autres vaisseaux périphériques.

Pour résoudre le problème posé de garantir un parfait contact du système de coupe par l'ablation des tissus calcifiés avec un résultat de décalcification optimal, le moyen de guidage réglable est un ruban souple apte à être déployé d'une manière circulaire en position de contact avec les tissus calcifiés.

A noter qu'il est possible d'ajuster l'expansion du ruban souple de façon à étendre progressivement la zone décalcifiée, toujours de manière circulaire. On observe également, qu'après avoir réalisé l'ablation des tissus, que la longueur du ruban déployé fournit une évaluation correcte du diamètre de la racine aortique, permettant le choix de la taille appropriée de l'implant TAVI.

Avantageusement, le ruban est déployé d'une manière excentrique par rapport à l'embout.

Dans une forme de réalisation, le ruban est monté en combinaison, d'une part, avec un arbre rotatif actionné par un organe de manoeuvre accessible à partir de l'extérieur du cathéter, et d'autre part, avec une partie fixe du cathéter à partir de laquelle est déployé ledit ruban sous l'effet d'une action exercée sur ledit organe de manoeuvre afin d'augmenter le diamètre du ruban jusqu'à ce qu'il vienne en contact avec la paroi de la valve aortique.

L'une des extrémités du ruban est fixée à l'arbre rotatif pour être enroulée sur ce dernier et déborder au travers d'une ouverture que présente le cathéter afin d'être fixée par son autre extrémité dans la partie dudit cathéter constituée par une fente afin de permettre audit ruban de déborder d'une manière excentrique.

Pour résoudre le problème posé de couper et enlever les feuillets calcifiés et les tissus environnants, le système de coupe présente deux têtes de coupe rotatives motorisées disposées co-axialement l'une au-dessus de l'autre, la tête située à l'extrémité de l'embout et intervenant en premier pour enlever les tissus calcifiés, présente des agencements aptes à réaliser une coupe grossière par broyage, tandis que l'autre tête présente des agencements aptes à réaliser une coupe fine par broyage. Les têtes de coupe débordent latéralement de l'embout d'une manière parallèle à ses génératrices.

Avantageusement, le moyen d'aspiration est synchronisé avec l'entraînement des têtes de coupe, les déchets de tissus étant évacués par au moins un conduit d'aspiration monté à l'intérieur du cathéter.

Selon d'autres caractéristiques, l'embout souple est réalisé à partir d'un matériau polymère du type silicone avec des marqueurs radio-opaque pour contrôler son positionnement dans la zone de travail.

Le dispositif comprend un système dynanométrique apte à mesurer le diamètre aortique où la valve doit être implantée sous une pression donnée, en position de maintien en contact du système de coupe avec les tissus calcifiés au moyen du ruban.

Comme indiqué, le dispositif décalcificateur peut être introduit directement dans la racine aortique avec une ponction directe de l'aorte ascendante ou transcathéter transfemoral ou autres, mais également, peut être mis en place au travers d'introducteurs de tous types connus et appropriés.

L'invention est exposée ci-après plus en détail à l'aide des figures des dessins annexés dans lesquels :
- la figure 1 est une vue en perspective avec coupe partielle montrant la partie active du dispositif transcathéter d'implantation,
- la figure 2 est à très grande échelle et à caractère schématique une vue en coupe transversale de l'embout pour l'ablation et l'aspiration des déchets avant déploiement du ruban positionneur
- la figure 3 est une vue correspondant à la figure 2, après déploiement du ruban positionneur,
- les figures 4 à 17 montrent les principales étapes pour une ablation transcathéter de tissus calcifiés au niveau des volets d'une valve aortique native au moyen du dispositif selon l'invention illustré aux figures 1, 2 et 3.

Le dispositif d'ablation est directement constitué d'un cathéter avec des dimensions permettant d'être utilisées avec ce type de procédure ou est inséré dans un cathéter introducteur de tous types connus et appropriés.

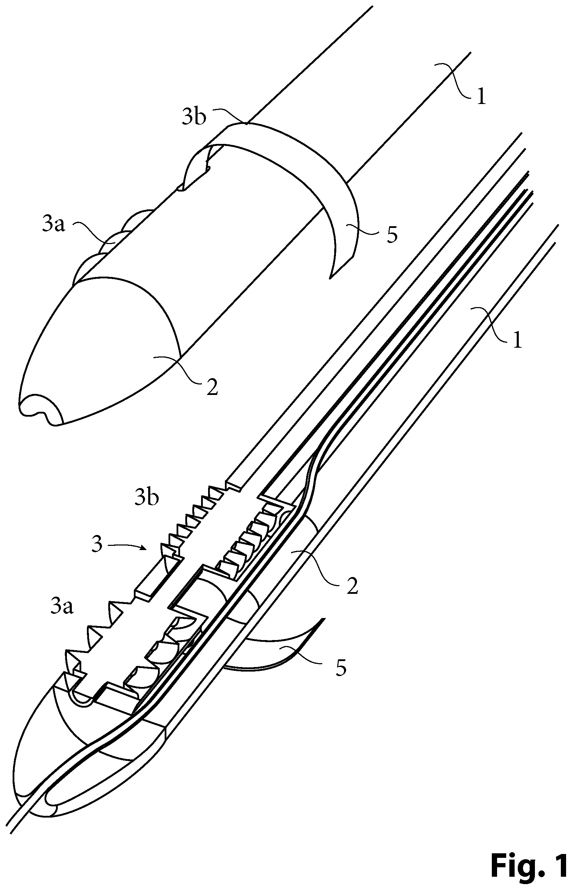

Comme illustré figure 1, notamment, le dispositif comprend un corps souple (1) faisant office de cathéter et présentant un embout (2) souple et flexible et conforme pour traverser les volets de la valve native calcifiée et au-dessus de la partie d'où doit être enlevée la calcification, ledit embout étant apte à coopérer avec un guide-fil (g). L'embout (2) présente au moins un système de coupe (3) monté en combinaison avec un moyen d'aspiration (4) sous-vide. L'embout (2) est, en outre, équipé d'un moyen de guidage réglable (5), apte à coopérer avec les tissus calcifiés, au fur et à mesure de l'opération d'ablation par le système de coupe (3), en combinaison avec un effet de trajectoire en spirale appliqué audit embout (2).

Comme cela est indiqué dans la suite de la description, l'ablation des tissus s'effectue selon une trajectoire en spirale partant du centre jusqu'à la périphérie de la valve par translation de rotation contre les bords des tissus par l'intermédiaire du système de coupe (3). La décalcification est effectuée en appliquant une pression latérale du système de coupe (3) en combinaison avec l'autre moyen de guidage (5) sur les tissus calcifiés, évitant de couper la paroi aortique une fois que la valve est complètement nettoyée.

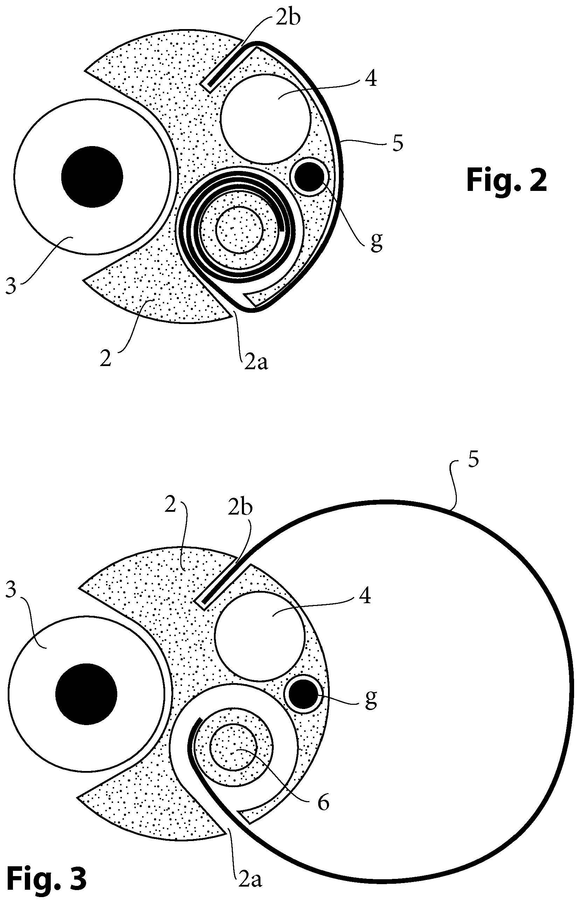

Le moyen de guidage (5) est constitué par un ruban souple apte à être déployé d'une manière circulaire en position de contact avec les tissus calcifiés. Avantageusement, le ruban (5) est déployé de manière excentrique par rapport à l'embout (2), figures 2 et 3. Dans une forme de réalisation, le ruban (5) est monté en combinaison, d'une part avec un arbre rotatif (6) commandé par un organe de manoeuvre accessible à partir de l'extérieur du cathéter (1), et, d'autre part, avec une partie fixe de l'embout (2) à partir de laquelle est déployé ledit ruban (5). Plus particulièrement, l'une des extrémités du ruban (5) est fixée à l'arbre rotatif (6) pour être enroulée sur ce dernier et déborder au travers d'une ouverture (2a) que présente l'embout (2) afin d'être fixée par son autre extrémité sur la partie fixe dudit embout. Cette partie fixe est constituée, dans l'exemple illustré, par une fente (2b) afin de permettre audit ruban de déborder d'une manière excentrée, figure 3.

Comme il sera décrit dans la suite de la description, ce ruban (5) permet de maintenir le système de coupe (3) en contact avec les tissus résiduels enlevés sous l'effet du déroulement excentrique dudit ruban et de son accroissement en diamètre, au fur et à mesure de la décalcification progressive en suivant une trajectoire en spirale. Etant donné que l'ablation des tissus progresse depuis le centre de la valve native jusqu'à sa périphérie, il est important d'avoir un certain contact entre le système de coupe et les tissus calcifiés.

Le système de coupe (3) présente deux têtes de coupe rotatives (3a) et (3b) motorisées et disposées co-axialement l'une au-dessus de l'autre. Les têtes de coupe (3a) (3b) débordent latéralement de l'embout (2) d'une manière parallèle à ses génératrices. La tête (3a), située à l'extrémité de l'embout (2), et en-dessous de la tête (3b) dans une position verticale du cathéter, pour intervenir en premier afin d'enlever les tissus calcifiés, présente une denture apte à réaliser une coupe grossière par broyage. Inversement, l'autre tête de coupe (3b), disposée au-dessus de la tête (3a), présente des agencements aptes à réaliser une coupe fine par broyage. Ces caractéristiques permettent à l'opérateur d'adapter la décalcification au type et à la densité de la valve native calcifiée par glissement et d'attendre les résultats en fonction de la valve à implanter.

Sans pour cela sortir du cadre de l'invention, d'autres systèmes de coupe, tels que des systèmes ultra soniques, peuvent être envisagés.

Le moyen d'aspiration est avantageusement synchronisé avec l'entraînement du système de coupe (3). Ce moyen d'aspiration, de tous types connus et appropriés, est assujetti à un conduit d'aspiration (4) monté à l'intérieur de l'embout (2) et du cathéter (1).

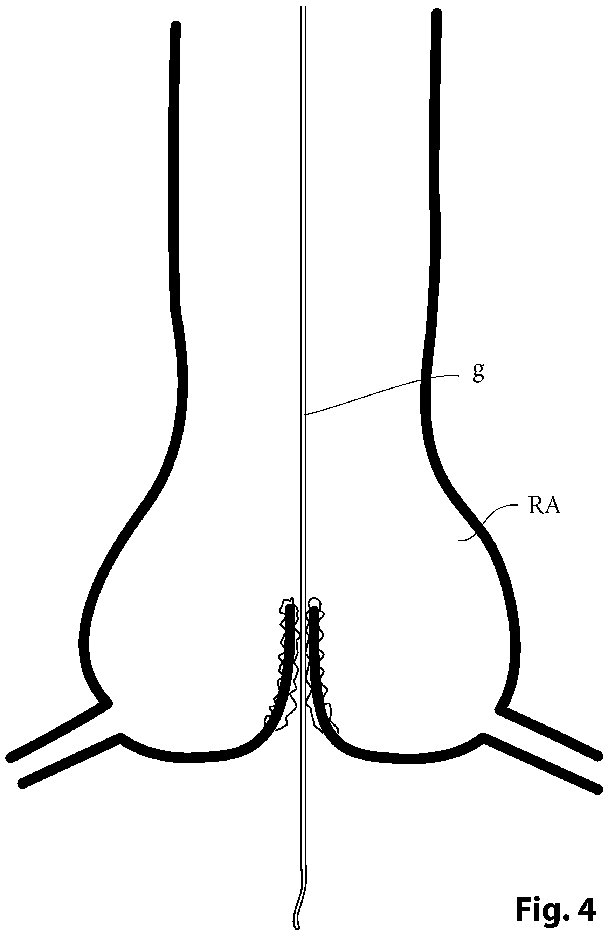

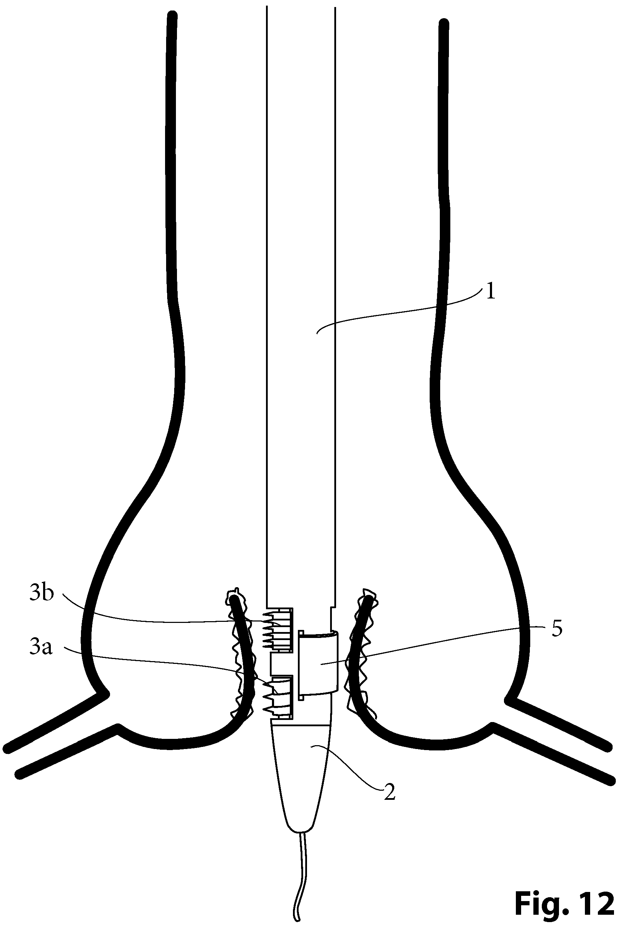

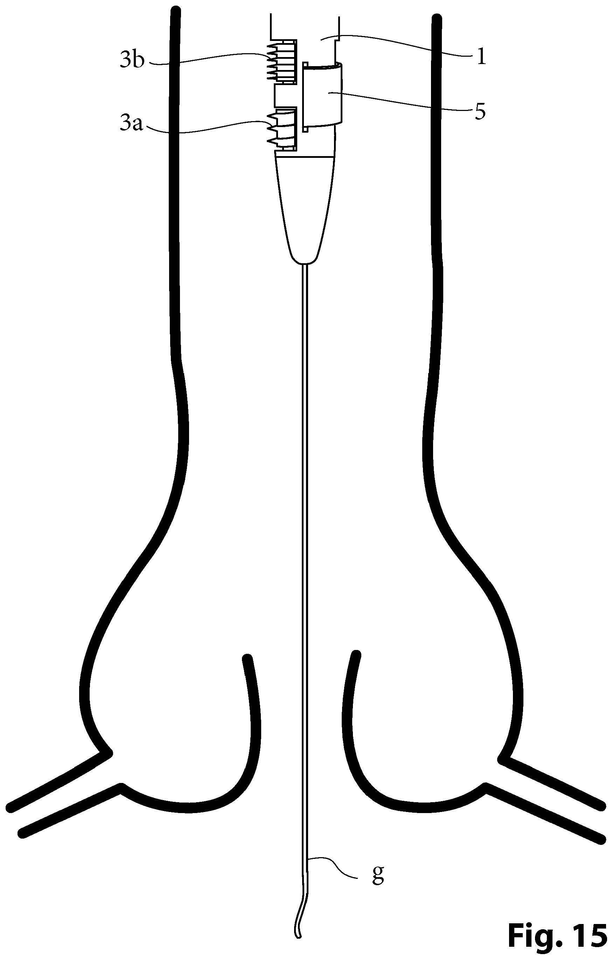

On se réfère aux figures 4 à 17 qui montrent les différentes séquences pour la mise en place du dispositif transcathéter et pour l'ablation des tissus calcifiés au niveau des volets de la valve aortique native. La figure 4 montre la racine aortique après mise en place guide-fil (g), qui, de manière connue, est engagé afin de traverser la valve aortique sténosée.

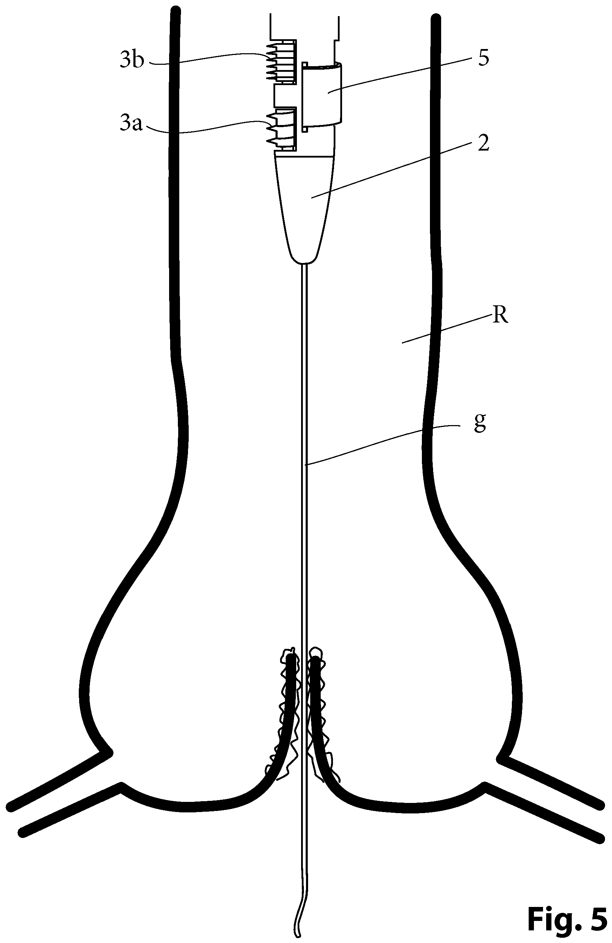

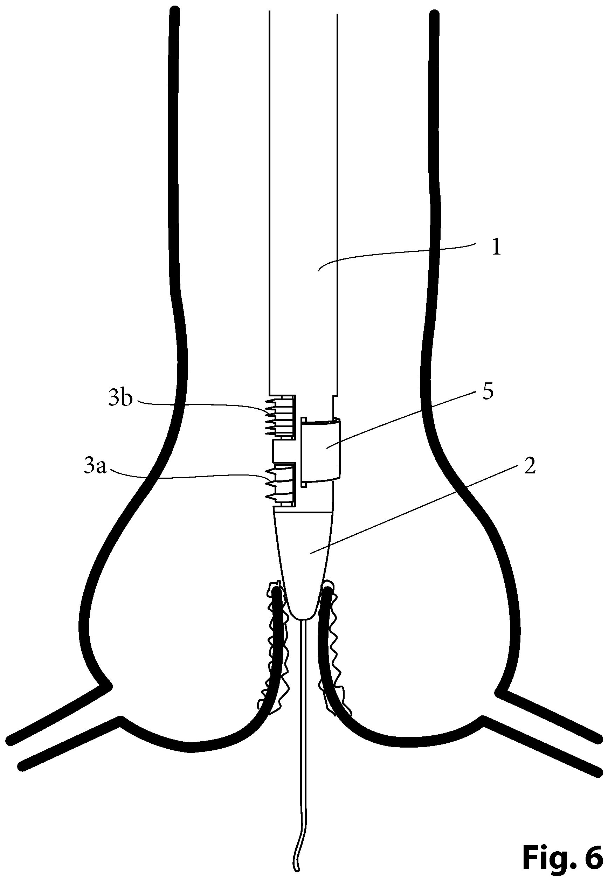

Le dispositif transcathéter sous forme d'un tuyau flexible (1), par exemple, avec son embout (2), est introduit en étant en relation avec le guide-fil (g) pour être dirigé au-dessus de la partie où doit être enlevée la calcification, figures 5 et 6. Un marquage radio-opaque identifie sous fluoroscopie et sous TEE la partie d'ablation sur le cathéter (1). Un marquage est placé au-dessus et un autre au-dessous de la valve aortique.

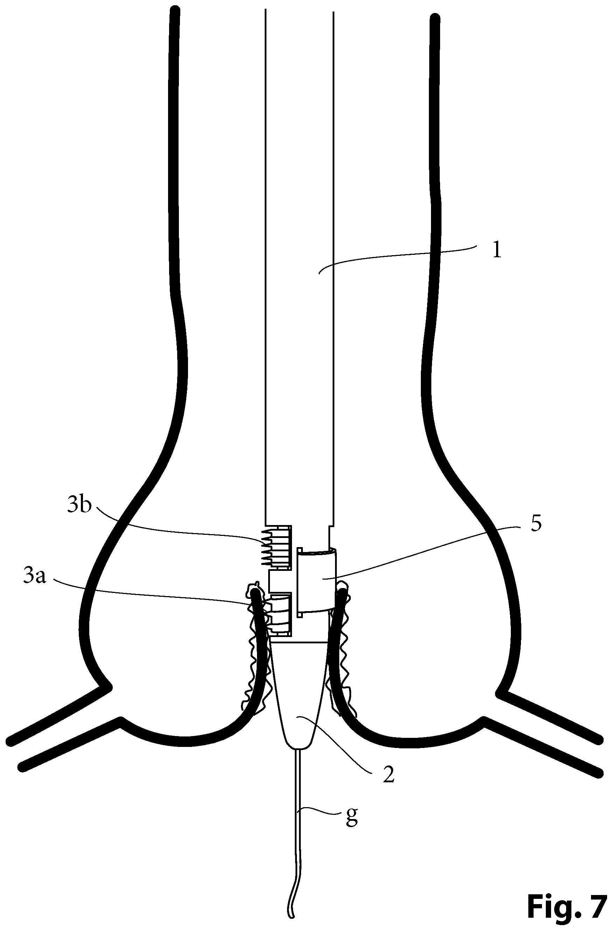

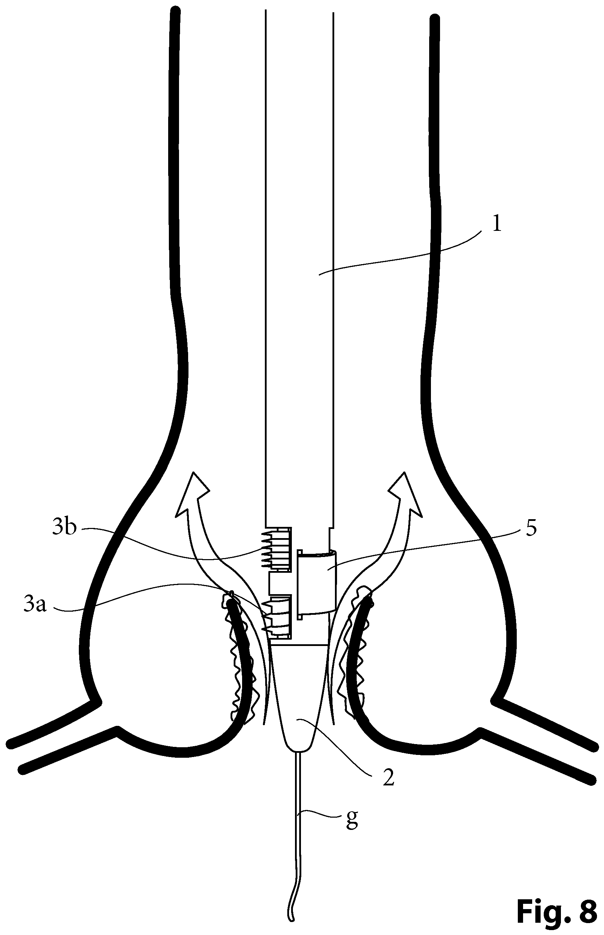

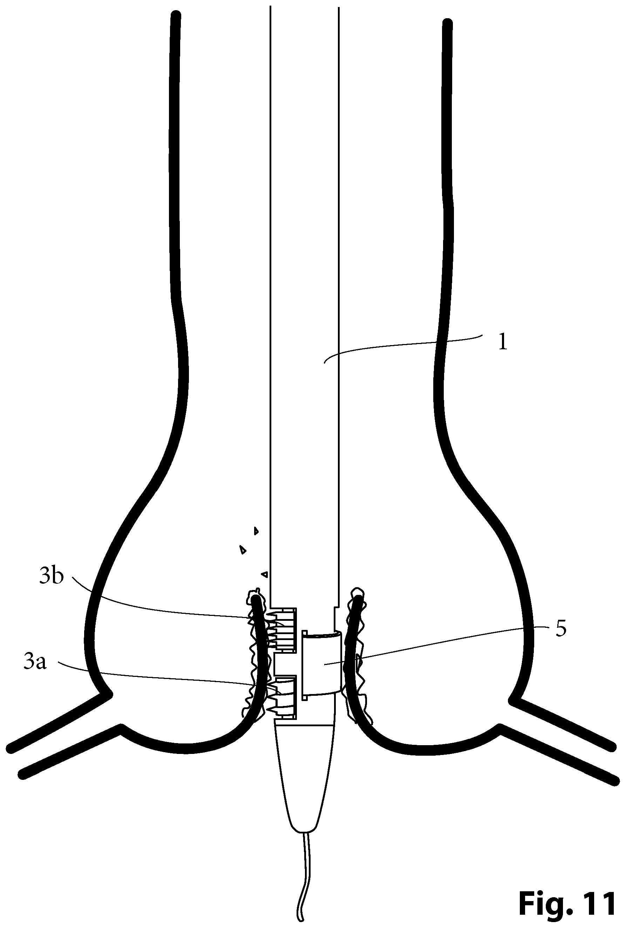

La procédure d'ablation en tant que telle peut commencer en utilisant d'abord la tête de coupe (3a) présentant la denture grossière, figures 7 et 8 puis la tête de coupe (3b) présentant la denture fine, figure 11.

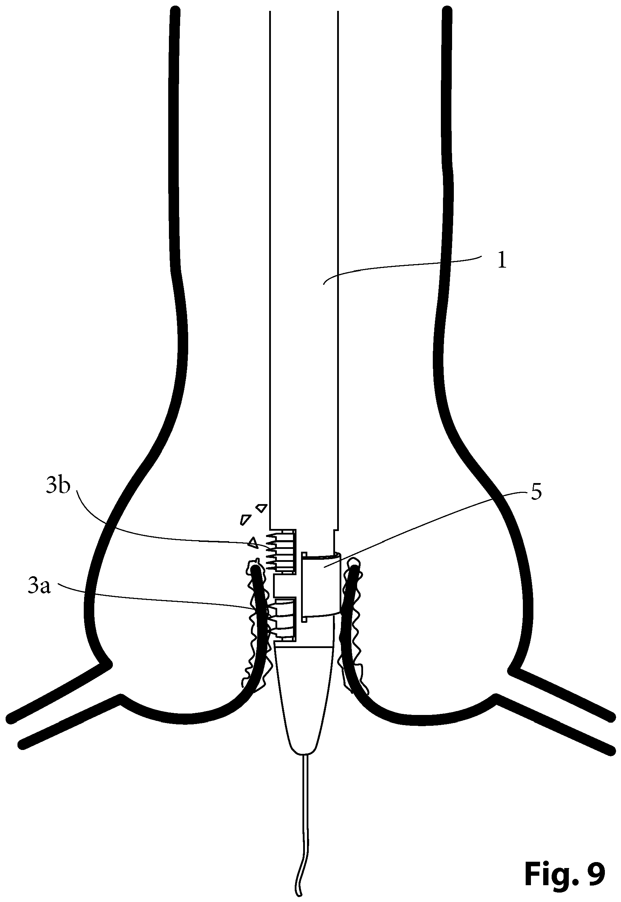

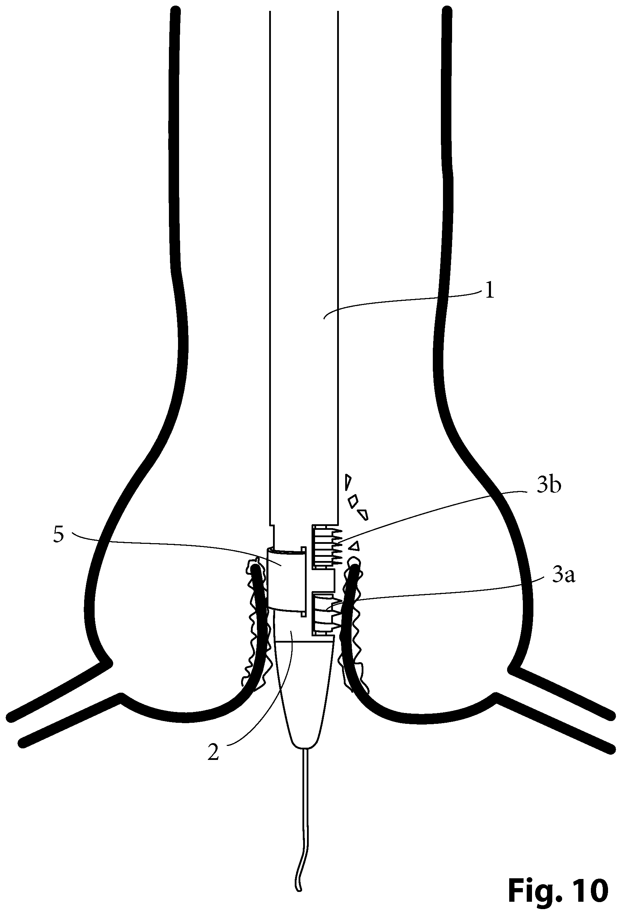

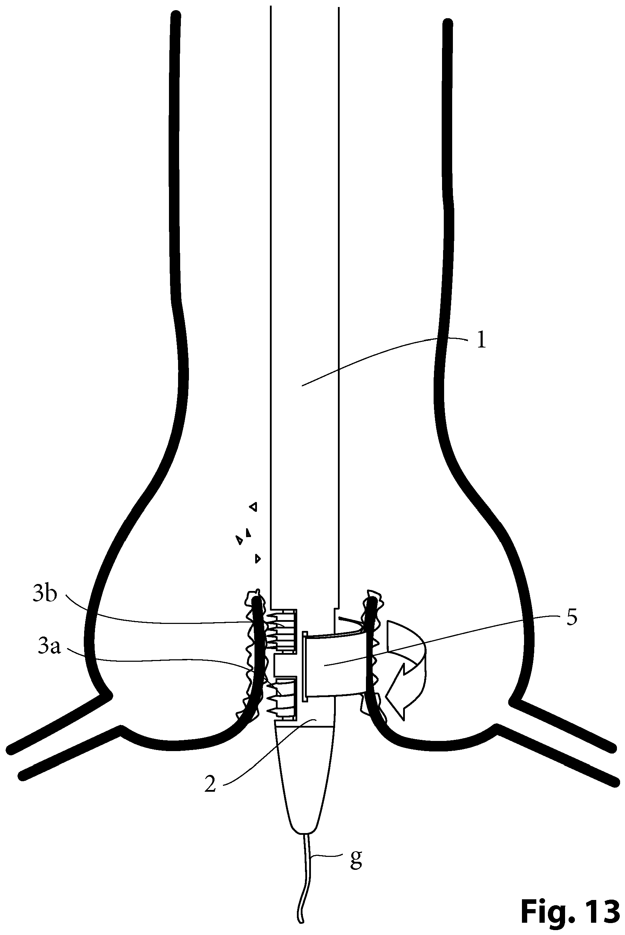

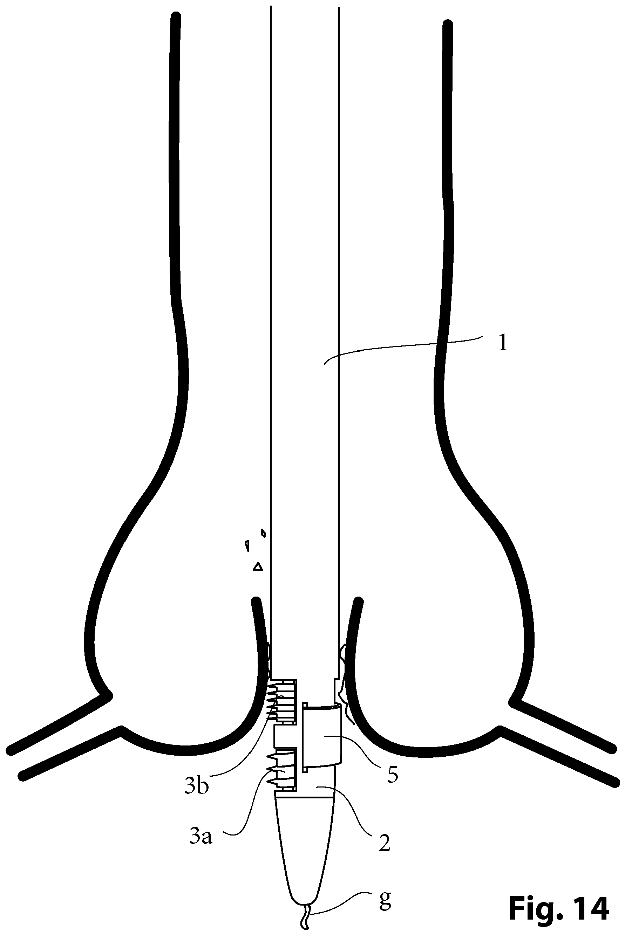

La procédure d'ablation des tissus calcifiés commence à partir du centre de la valve native, figure 9, et progresse via une trajectoire en spirale avec une rotation progressive de la tête d'ablation, (figures 10 et 11), en combinaison avec le ruban (5) qui est déployé de manière excentrée afin de maintenir un contact continu de la tête de coupe (3) avec les tissus calcifiés (figure 13). La tête de coupe à denture fine (3b) peut ensuite être utilisée pour enlever les tissus calcifiés, (figure 14).

Comme indiqué, pendant la procédure de décalcification, le système d'aspiration (4) est mis en service afin d'être synchronisé avec le système de coupe (3) pour évacuer les débris de calcium et les fibres des feuillets de la valve native au moyen du conduit d'aspiration situé à l'intérieur du cathéter (1).

Cette aspiration est particulièrement importante étant donné que l'intervention s'effectue en circulation sanguine et non pas en circulation extra-corporelle.

La procédure de décalcification en tant que telle peut être poursuivie jusqu'à ce que les feuillets natifs soient suffisamment amincis ou jusqu'à obtenir une complète ablation des feuillets afin de laisser une racine aortique nette en vue d'une implantation d'une prothèse de valve aortique spécifique.

De manière avantageuse, lorsque la procédure d'ablation de calcium est terminée, l'opérateur peut mesurer le diamètre de la racine aortique par le ruban (5). De fait, ce ruban peut être équipé d'un système de mesure et d'un dynamomètre qui permet de mesurer le diamètre de la racine aortique sous pression spécifique, la lecture étant visible à partir de l'extérieur du cathéter. Le fait que cette racine aortique soit sous pression spécifique est important étant donné que cette condition donne des informations nécessaires à l'opérateur sur l'élasticité résiduelle de l'anneau aortique, partiellement ou totalement décalcifié, afin de choisir la bonne taille et la bonne prothèse valvulaire.

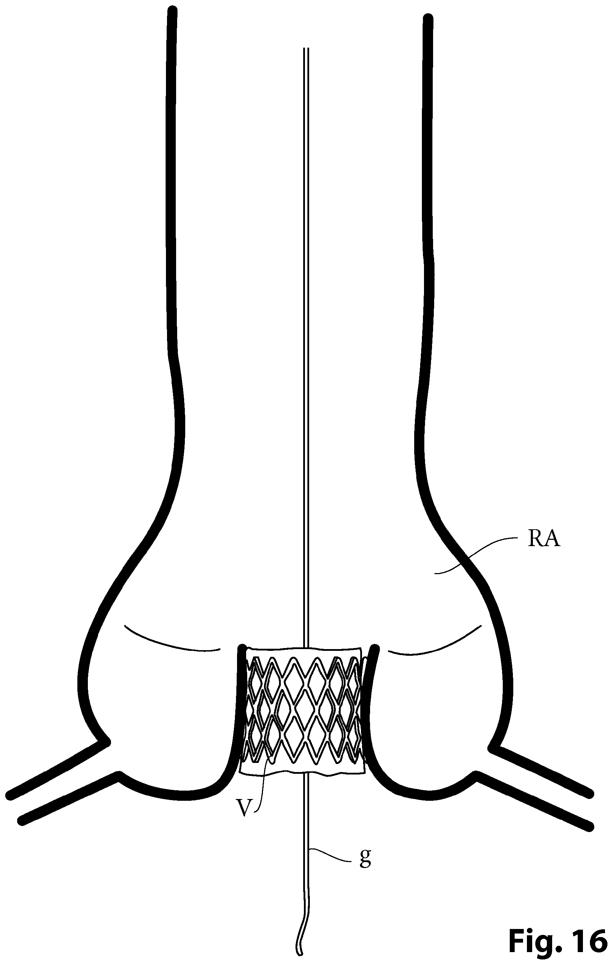

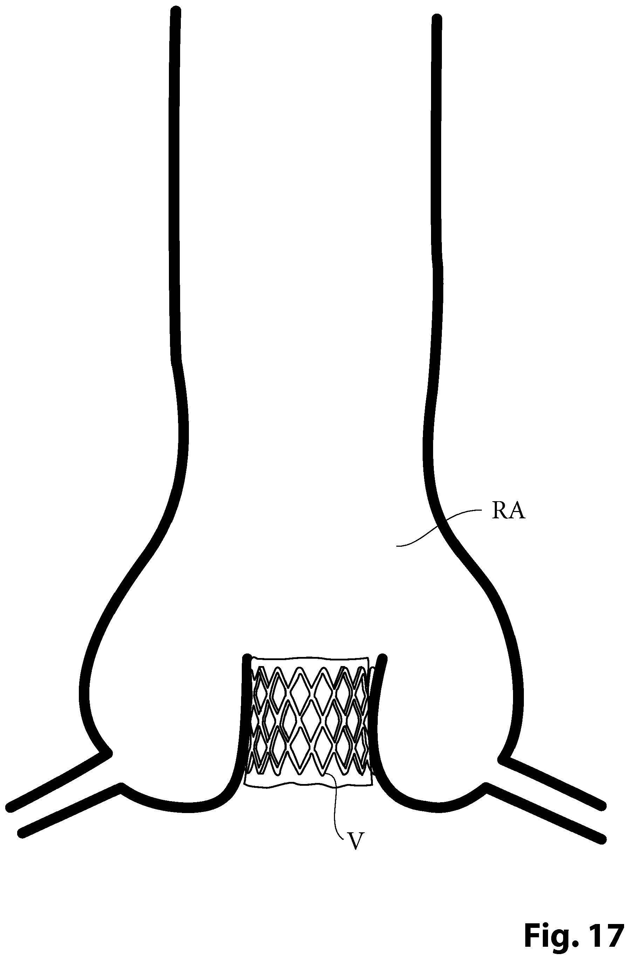

Après avoir terminé l'opération de décalcification, le cathéter est enlevé, figure 16, la valve TAVI est mise en place, figures 16 et 17, au moyen d'un introducteur.

Les avantages ressortent bien de la description.

## Revendications

1. Dispositif transcathéter pour l'ablation de tissus calcifiés au niveau des volets d'une valve aortique, qui comprend un corps souple (1) faisant office de cathéter et présentant un embout souple et flexible (2) coopérant avec un guide-fil (g) préalablement introduit et apte à traverser les volets de la valve au-dessus de la partie où doivent être enlevés les tissus calcifiés, ledit embout (2) présentant au moins un système de coupe (3) étant monté en combinaison avec un moyen d'aspiration sous-vide (4), **caractérisé en ce que** ledit embout (2) étant équipé d'un moyen de guidage réglable (5) apte à coopérer avec les tissus calcifiés au fur et à mesure de l'opération d'ablation par le système de coupe (3) en combinaison avec un effet de trajectoire en spirale appliqué à l'embout, et **en ce que**
le système de coupe (3) comprenant deux têtes de coupe rotatives motorisées (3a) et (3b) disposées co-axialement l'une au-dessus de l'autre, la tête (3a) située à l'extrémité de l'embout et intervenant en premier pour enlever les tissus calcifiés, présente des agencements aptes à réaliser une coupe grossière par broyage, tandis que l'autre tête (3b) présente des agencements aptes à réaliser une coupe fine par broyage.

2. Dispositif selon la revendication 1, ***caractérisé* en ce que** le moyen de guidage réglable est un ruban souple (5) apte à être déployé d'une manière circulaire en position de contact avec les tissus calcifiés.

3. Dispositif selon la revendication 2, ***caractérisé* en ce que** le ruban (5) est déployé d'une manière excentrique par rapport à l'embout (2).

4. Dispositif selon la revendication1, ***caractérisé* en ce que** les têtes de coupe (3a) et (3b) débordent latéralement de l'embout d'une manière parallèle à ses génératrices.

5. Dispositif selon la revendication 1, ***caractérisé* en ce que** le moyen d'aspiration est synchronisé avec l'entraînement des têtes de coupe, les déchets de tissus étant évacués par au moins un conduit d'aspiration (4) monté à l'intérieur du cathéter.

6. Dispositif selon la revendication 2, ***caractérisé* en ce que** le ruban (5) est monté en combinaison, d'une part, avec un arbre rotatif (6) actionné par un organe de manoeuvre accessible à partir de l'extérieur du cathéter, et d'autre part, avec une partie fixe de l'embout à partir de laquelle est déployé ledit ruban (5) sous l'effet d'une action exercée sur ledit organe de manoeuvre afin d'augmenter le diamètre du ruban jusqu'à ce qu'il vienne en contact avec la paroi de la valve aortique.

7. Dispositif selon la revendication 6, ***caractérisé* en ce que** l'une des extrémités du ruban (5) est fixée à l'arbre rotatif pour être enroulée sur ce dernier et déborder au travers d'une ouverture (2a) que présente l'embout afin d'être fixée par son autre extrémité dans la partie fixe dudit embout constituée par une fente (2b) afin de permettre audit ruban de déborder d'une manière excentrique.

8. Dispositif selon la revendication 1, ***caractérisé* en ce que** l'embout souple (2) est réalisé à partir d'un matériau polymère du type silicone avec marqueur radio-opaque pour contrôler son positionnement dans la zone de travail.

9. Dispositif selon la revendication 2, ***caractérisé* en ce qu'**il comprend un système dynanométrique apte à mesurer le diamètre aortique où la valve doit être implantée sous une pression donnée, en position de maintien en contact du système de coupe avec les tissus calcifiés au moyen du ruban (5).

## Patentansprüche

1. Transkathetervorrichtung zur Ablation von verkalktem Gewebe auf der Höhe der Taschen einer Aortenklappe, welche einen nachgiebigen Körper (1) umfasst, der als Katheter dient und eine nachgiebige und flexible Spitze (2) aufweist, welche mit einer Drahtführung (g) zusammenwirkt, die zuvor eingeführt wurde und geeignet ist, die Taschen der Klappe über dem Teil zu durchqueren, wo das verkalkte Gewebe entfernt werden muss, wobei die Spitze (2) mindestens ein Schneidsystem (3) aufweist, das in Kombination mit einem Vakuumabsaugmittel (4) montiert ist, **dadurch gekennzeichnet, dass** die Spitze (2) mit einem regulierbaren Führungsmittel (5) ausgestattet ist, das geeignet ist, mit dem verkalkten Gewebe während des Ablationsvorgangs durch das Schneidsystem (3) in Kombination mit einem Effekt einer Spiralbahn zusammenzuwirken, der auf die Spitze ausgeübt wird, und dadurch, dass das Schneidsystem (3) zwei motorisierte rotierende Schneidköpfe (3a) und (3b) umfasst, die koaxial zueinander angeordnet sind, wobei der Kopf (3a), der am Ende der Spitze angeordnet ist und als Erster die Entfernung des verkalkten Gewebes vornimmt, Anordnungen aufweist, die geeignet sind, einen groben Schnitt durch Schleifen vorzunehmen, während der andere Kopf (3b) Anordnungen aufweist, die geeignet sind, einen feinen Schnitt durch Schleifen vorzunehmen.

2. Vorrichtung nach Anspruch 1, **dadurch gekennzeichnet, dass** das regulierbare Führungsmittel ein nachgiebiges Band (5) ist, das geeignet ist, kreisförmig in eine Kontaktposition mit dem verkalkten Gewebe gebracht zu werden.

3. Vorrichtung nach Anspruch 2, **dadurch gekennzeichnet, dass** das Band (5) exzentrisch in Bezug auf die Spitze (2) aufgebracht wird.

4. Vorrichtung nach Anspruch 1, **dadurch gekennzeichnet, dass** die Schneidköpfe (3a) und (3b) lateral die Spitze parallel zu ihren Erzeugenden begrenzen.

5. Vorrichtung nach Anspruch 1, **dadurch gekennzeichnet, dass** das Absaugmittel mit dem Antrieb der Schneidköpfe synchronisiert ist, wobei Gewebeabfälle durch mindestens eine Absaugleitung (4) abgeführt werden, die im Inneren des Katheters montiert ist.

6. Vorrichtung nach Anspruch 2, **dadurch gekennzeichnet, dass** das Band (5) in Kombination einerseits mit einer Drehwelle (6), die von einem Manövrierelement betätigt wird, das von außerhalb des Katheters zugänglich ist, und andererseits mit einem festen Teil der Spitze montiert ist, von der das Band (5) unter dem Effekt einer Wirkung aufgebracht wird, die auf das Manövrierelement ausgeübt wird, um den Durchmesser des Bands zu erhöhen, bis es mit der Wand der Aortenklappe in Kontakt gelangt.

7. Vorrichtung nach Anspruch 6, **dadurch gekennzeichnet, dass** eines der Enden des Bands (5) an der Drehwelle befestigt ist, um um diese Letztere gewunden zu werden und quer durch eine Öffnung (2a) zu verlaufen, welche die Spitze aufweist, um an seinem anderen Ende an dem festen Teil der Spitze befestigt zu werden, der durch einen Schlitz (2b) gebildet wird, um es dem Band zu gestatten, auf exzentrische Weise zu verlaufen.

8. Vorrichtung nach Anspruch 1, **dadurch gekennzeichnet, dass** die nachgiebige Spitze (2) aus einem Polymermaterial vom Silikontyp mit einer strahlenundurchlässigen Markierung gebildet ist, um ihre Positionierung in der Arbeitszone zu steuern.

9. Vorrichtung nach Anspruch 2, **dadurch gekennzeichnet, dass** diese ein dynamometrisches System umfasst, das geeignet ist, den Aortendurchmesser zu messen, wo die Klappe unter einem gegebenen Druck implantiert werden muss, in einer Position zum Halten des Schneidsystems in Kontakt mit dem verkalkten Gewebe durch das Band (5).

## Claims

1. Transcatheter device for the ablation of calcified tissue at the flaps of an aortic valve which it comprises a soft body (1) serving as a catheter and having a soft and flexible endpiece (2) engaging with a guide wire (g) previously introduced and suitable for passing through the flaps of the valve above the part where the calcified tissue needs to be removed, said endpiece (2) having at least one cutting system (3) mounted in combination with a vacuum suction means (4), **characterized in that** said endpiece (2) is equipped with an adjustable guide means (5) suitable for engaging with the calcified tissue over the course of the ablation operation performed by the cutting system (3) in combination with a spiral path effect applied to the endpiece, and **in that** the cutting system (3) comprising two motorized rotary cutting heads (3a) and (3b) arranged coaxially one above the other, the head (3a) located at the end of the endpiece, and serving first to remove the calcified tissue, has arrangements suitable for performing a rough cut by grinding, while the other head (3b) has arrangements suitable for performing a fine cut by grinding.

2. Device according to Claim 1, **characterized in that** the adjustable guide means is a soft band (5) suitable for being deployed in a circular manner in the position of contact with the calcified tissue.

3. Device according to Claim 2, **characterized in that** the band (5) is deployed in an eccentric manner with respect to the endpiece (2).

4. Device according to Claim 1, **characterized in that** the cutting heads (3a) and (3b) protrude laterally from the endpiece in a manner parallel to the generatrices thereof.

5. Device according to Claim 1, **characterized in that** the suction means is synchronized with the driving of the cutting heads, the tissue debris being evacuated via at least one suction conduit (4) mounted inside the catheter.

6. Device according to Claim 2, **characterized in that** the band (5) is mounted in combination, on the one hand, with a rotary shaft (6) actuated by a maneuvering element accessible from outside the catheter, and, on the other hand, with a stationary part of the endpiece from which said band (5) is deployed under the effect of an action exerted on said maneuvering element in order to increase the diameter of the band until it comes into contact with the wall of the aortic valve.

7. Device according to Claim 6, **characterized in that** one of the ends of the band (5) is fixed to the rotary shaft so as to be wound around the latter and protrude through an opening (2a) of the endpiece, in order to be fixed by its other end in the stationary part of said endpiece formed by a slit (2b), so as to allow said band to protrude in an eccentric manner.

8. Device according to Claim 1, **characterized in that** the soft endpiece (2) is made from a polymer material such as silicone, with a radiopaque marker for monitoring its position in the operating zone.

9. Device according to Claim 2, **characterized in that** it comprises a dynamometric system suitable for measuring the aortic diameter where the valve is to be implanted under a given pressure, in a position in which the cutting system is maintained in contact with the calcified tissue by means of the band (5).
